(19) Europäisches Patentamt
European Patent Office
Office européen des brevets

(11) Veröffentlichungsnummer: **0 124 831**
**B1**

(12) # EUROPÄISCHE PATENTSCHRIFT

(45) Veröffentlichungstag der Patentschrift: **15.10.86**

(51) Int. Cl.⁴: **C 07 C 139/00**, C 07 C 143/68

(21) Anmeldenummer: **84104731.9**

(22) Anmeldetag: **27.04.84**

(54) **Verfahren zur Herstellung von O-Sulfonsäureestern gegebenenfalls substituierter Aminonaphthole.**

(30) Priorität: **10.05.83 DE 3316984**

(43) Veröffentlichungstag der Anmeldung: **14.11.84 Patentblatt 84/46**

(45) Bekanntmachung des Hinweises auf die Patenterteilung: **15.10.86 Patentblatt 86/42**

(84) Benannte Vertragsstaaten: **CH DE FR GB IT LI**

(56) Entgegenhaltungen:
**DE-C-193 099**
**DE-C-254 715**
**US-A-2 518 023**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Henk, Hermann, Dr., Roggendorfstrasse 55, D-5000 Koeln 80 (DE)**
Erfinder: **Vater, Hans- Joachim, Dr., Klaus- Groth-Strasse 2, D-2220 St. Michaelisdonn (DE)**

**EP 0 124 831 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

O-Sulfonsäureester von Aminonaphtholen bzw. Aminonaphtholsulfonsäuren sind seit langem bekannte Zwischenprodukte für Farbstoffe.

Ihre Herstellung erfolgt durch "Einwirkung von Sulfonsäurechloriden auf eine bis zum Schluß der Reaktion ätzalkalisch gehaltene Lösung des Aminonaphthols bzw. siener Sulfonsäuren" (DRP 202 116, Frdl. *9*, 388).

Dieses Verfahren hat den Nachteil, daß die Veresterung unter den genannten Bedingungen nicht selektiv verläuft. Man erhält stets eine gewisse Menge des an der Aminogruppe acylierten Produktes, welches einerseits die Ausbeute mindert, andererseits zu unreinen Farbstoffen führt.

Gegenstand der vorliegenden Erfindung ist ein verbessertes Verfahren zur Herstellung von Estern der Formel

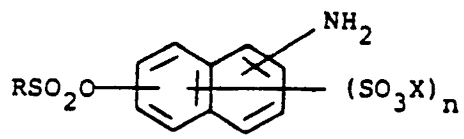
(I)

worin

X = H oder einwertiges Kation, insbesondere Alkalimetallkation,
R = Alkyl, Aralkyl oder Aryl
n = 1 oder 2

dadurch gekennzeichnet, daß man zunächst ein Aminonaphthol der Formel

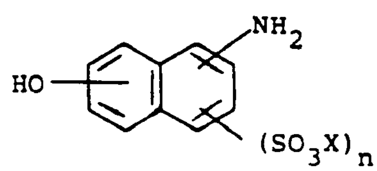
(II)

in eine N-Acylaminoverbindung der Formel

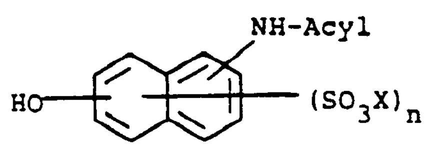
(III)

umwandelt, diese anschließend mit Sulfonsäurehalogeniden der Formel

$$R\ SO_2Y \qquad (IV)$$

worin

Y = Halogen, vorzugsweise C1

zu den Verbindungen

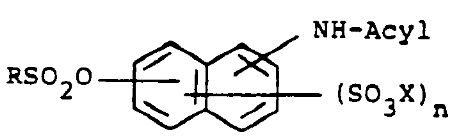
(V)

umsetzt und anschließend den N-Acylrest abspaltet.

Die gesamte Reaktion erfolgt im wäßrigen Milieu und wird vorzugsweise im "Eintopf-", d.h. ohne Zwischenisolierungen durchgeführt. Die Acylierung der Aminogruppe erfolgt dabei im pH Bereich von 3 bis 11, bevorzugt 5 bis 9.

Die Veresterung der Hydroxylgruppe erfolgt im pH Bereich von 7 bis 12, bevorzugt 7 bis 10. Die N-Acrylierung und O-Veresterung erfolgen im Temperaturbereich von 0—80°C, bevorzugt bei 10—60°C, besonders bevorzugt bei 20—40°C. die Abspaltung der N-Acrylgruppe verläuft unter mineralsauren Bedingungen, d.h. bei pH-Werten <2 und Temperaturen von etwa 50—100°C, bevorzugt bei pH <1 und Temperaturen von 60—100°C.

Geeignete Reste R sind beispielsweise $C_1$-$C_4$-Alkylreste die weitere Substituenten beispielsweise Chlor enthalten können, gegebenenfalls substituierte Phenyl-und Phenyl-$C_1$-$C_4$-alkylreste.

Geeignete Kationen sind insbesondere Natrium-Kationen.

Als Ausgangsverbindungen II kommen beispielsweise in Frage:

8-Amino-1-naphthol-3-disulfonsäure,
8-Amino-1-naphthol-3,5-disulfonsäure,
8-Amino-1-naphthol-5-sulfonsäure,
6-Amino-1-naphthol-4,8-disulfonsäure,
6-Amino-1-naphthol-3-sulfonsäure,
7-Amino-1-naphthol-3-sulfonsäure,
8-Amino-1-naphthol-3-sulfonsäure,
5-Amino-1-naphthol-3-sulfonsäure,
6-Amino-2-naphthol-4-sulfonsäure,
8-Amino-2-naphthol-6-sulfonsäure,

Geeignete Acylierungsmittel zum intermediären Schutze der Aminogruppe sind die Halogenide oder Anhydride von aliphatischen oder aromatischen Mono- oder Polycarbonsäuren, sowie ein Halogenid heterocyclischer Säuren.

Beispielhaft seien gennant:

Benzoylchlorid,
Benzoesäureanhydrid,
2-3- oder 4-Chlorbenzensoesäurechlorid,
2-,5-Dichlobenzoesäurechlorid,
3- oder 4-Nitrobenzoylchlorid,
o- m- oder p-Tolylsäurechlorid,
Phthaloyl-, Isophtahaloyl- oder Terephthaloylchlorid,
Bernsteinsäureanhydrid,
Glutarsäureanhydrid,
Maleininsäureanhydrid,
Dichlormaleinsäureanhydrid,
Phthalsäureanhydrid,
Naphthalsäureanhydrid,
Acetylchlorid,
Chloracetylchlorid,
Acetanhydrid,
Dichloracetylchlorid,
Bromacetylchlorid,
Propionsäurechlorid,
Propionsäureanhydrid,
3-Chlorpropionsäurechlorid,
Buttersäurechlorid,
Buttersäureanhydrid,
4-Chlorbuttersäurechlorid,
Isovalerylchlorid,
Stearoylchlorid,
Methacrylsäurechlorid,
Ölsäurechlorid,
Oxalylchlorid,
Succinylchlorid,
Sebacinoylchorid,
Phosgen,
Cyanurchlorid,
Tetrachlorpyrimidin,
Dichlorchinoxalin.

Bevorzugte Acylierungsmittel sind Anhydride zweibasischer Säuren, insbesonder cyclische Anhydride aliphatischer oder aromatischer Dicarbonsäure wie

Maleinsäureanhydrid,
Dichlormaleinsäureanhydrid,
Bersteinsäureanhydrid,
Methylbernsteinsäureanhydrid,
Citraconsäureanhydrid,
Pthalsäureanhydrid,
Naphthalsäureanhydrid,

Geeignete Sulfonsäurehalogenide zur Veresterung der Hydroxylgruppe sind beispielsweise:

Mesylchlorid,
2-Chlorethansulfonsäurechlorid,
Butan-1-sulfonsäurechlorid,
4-Chlorbutan-1-sulfonsäurechlorid,
Benzolsulfochlorid,
3- oder 4-Chlorbenzolsulfochlorid,
2-,3- oder 4-Nitrobenzolsulfonsäurechlorid,
o- oder p-Toluolsulfonsäurechlorid,
1,3-Benzoldisulfonsäuredichlorid,
Acetanilid-4-sulfonsäurechlorid.

Bevorzugt sind
Mesylchlorid, Benzolsulfochlorid und p-Toluolsulfochlorid.

Beispiel 1

494 g 8-Amino-1-naphthol-3,6-disulfonsäure werden in ca. 1000 ml Wasser alkalisch gelöst, mit 180 g Maleinsäureanhydrid versetzt. Gleichzeitig wird mit ca. 300 ml einer 40%igen Natronlauge der pH-Wert alkalisch gehalten. Nach ca. 2 Stunden ist die N-Acylierung beendet, was sich chromatographisch verfolgen läßt. Zu der erhaltenen Lösung tropft man langsam ca. 220 ml Benzolsulfonsäurechlorid. Gleichzeitig hält man mit weiteren 130 ml einer 40%igen Natronlauge den pH-Wert stets schwach alkalisch. Die Temperatur steigt geringfügig. Die erhaltene Lösung wird mit ca. 700 ml einer 30%igen Salzsäure versetzt un ca. 1 Stunde bei 90°C gerührt. Man erhalt eine Supension von 8-Amino-1-benzolsulfonyloxinaphthalin-3,6-disulfonsäure in einem Volumen von ca. 3200 ml mit sehr hoher Reinheit und einer Ausbeute von ca. 95%. Diese Suspension kann direkt zur weiteren Farbstoffsynthese eingesetzt werden.

Ersetzt man 8-Amino-1-napthol-3,6-disulfonsäure aus Beispiel 1 durch die Edukte aus Tabelle I ferner Maleinsäureanhydrid aus Beispiel 1 durch entsprechende Acylierungsmittel aus Tabelle I und Benzolsulfonchlorid aus Beispiel 1 durch entsprechende Sulfonsäurechloride aus Tabelle I und verfährt im übrigen nach den Bedingungen aus Beispiel 1 so erhält man in hohen Ausbeuten und Reinheiten entsprechende Produkte aus Tabelle I, die ebenfalls wertvolle Zwischenprodukte für Farbstoffe darstellen.

Tabelle I

| Beispiel | Ausgangsprodukt Edukt | N-Acylierungsmittel | Sulfonsäurechlorid | Endprodukt |
|---|---|---|---|---|
| 2 | 8-Amino-1-naphthol-3,6-disulfonsäure | Acetanhydrid | Mesylchlorid | 8-Amino-1-methylsulfonyloxi-naphthalin-3,6-disulfonsäure |
| 3 | 8-Amino-1-naphthol-3,5-disulfonsäure | Maleinsäureanhydrid | Benzolsulfochlorid | 8-Amino-1-benzolsulfonyloxi-naphthalin-3,5-disulfonsäure |
| 4 | 8-Amino-1-naphthol-3,5-disulfonsäure | Phthalsäureanhydrid | Benzolsulfochlorid | 8-Amino-1-benzolsulfonyloxi-naphthalin-3,5-disulfonsäure |
| 5 | 8-Amino-1-naphthol-5-sulfonsäure | Benzoylchlorid | Benzolsulfochlorid | 8-Amino-1-benzolsulfonyloxi-naphthalin-5-sulfonsäure |
| 6 | 6-Amino-1-naphthol-4,8-disulfonsäure | Maleinsäureanhydrid | Benzolsulfochlorid | 6-Amino-1-benzolsulfonyloxi-naphthalin-4,8-disulfonsäure |
| 7 | 6-Amino-1-naphthol-4,8-disulfonsäure | Acetanhydrid | Benzolsulfochlorid | 6-Amino-1-benzolsulfonyloxi-naphthalin-4,8-disulfonsäure |
| 8 | 6-Amino-1-naphthol-3-sulfonsäure | Maleinsäureanhydrid | Benzolsulfochlorid | 6-Amino-1-benzolsulfonyloxi-naphthalin-3-sulfonsäure |
| 9 | 6-Amino-1-naphthol-3-sulfonsäure | Phosgen | Benzolsulfochlorid | 6-Amino-1-benzolsulfonyloxi-naphthalin-3-sulfonsäure |
| 10 | 6-Amino-1-naphthol-3-sulfonsäure | Phosgen | Mesylchlorid | 6-Amino-1-methylsulfonyloxi-naphthalin-3-sulfonsäure |
| 11 | 7-Amino-1-naphthol-3-sulfonsäure | Acetanhydrid | Benzolsulfochlorid | 7-Amino-1-benzolsulfonyloxi-naphthalin-3-sulfonsäure |
| 12 | 7-Amino-1-naphthol-3-sulfonsäure | Phosgen | Benzolsulfochlorid | 7-Amino-1-benzolsulfonyloxi-naphthalin-3-sulfonsäure |
| 13 | 5-Amino-1-naphthol-3-sulfonsäure | Maleinsäureanhydrid | Benzolsulfochlorid | 5-Amino-1-benzolsulfonyloxi-naphthalin-3-sulfonsäure |

**Patentansprüche**

1. Verfahren zur Herstullung von Estern der Formel

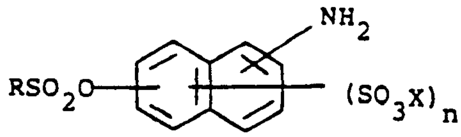

worin
X = H oder einwertiges Kation,
R = Alkyl, Aralkyl oder Aryl und
n = 1 oder 2,

dadurch gekennzeichnet, daß man zunächst Aminonaphthole der Formel

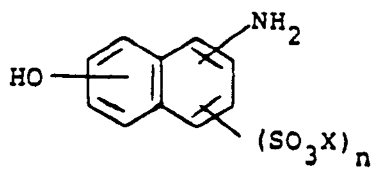

in eine N-Acylaminoverbindung der Formel

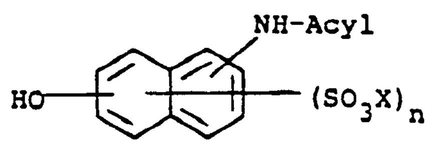

umwandelt, diese anschließend mit Sulfonsäurehalogeniden der Formel

R-$SO_2$Y

worin
Y = Halogen, vorzugsweise C1
zu den Verbindungen

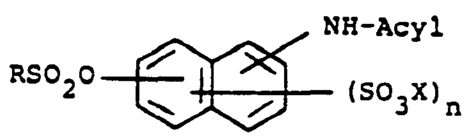

umsetzt und anschließend den N-Acylrest abspaltet.

2. Verfahren nach Anspruch 1, dadurch gekennzeichnet, daß zur N-Acylierung Anhydride zweibasischer aliphatischer oder aromatischer Carbonsäuren verwendet werden.

3. Verfahren nach Ansprüche 1 und 2, dadurch gekennzeichnet daß, ohne Zwischenisolierung gearbeitet wird.

**Revendications**

1. Procédé de production d'esters de formule

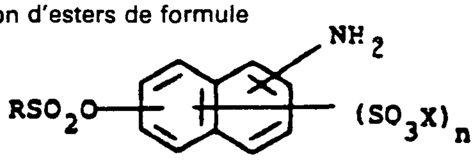

dans laquelle
X représente H ou un cation monovalent,
R représente un groupe alkyle, aralkyle ou aryle et
n a la valeur 1 ou 2,
caractérisé en ce qu'on transforme tout d'abord des aminonaphtols de formule

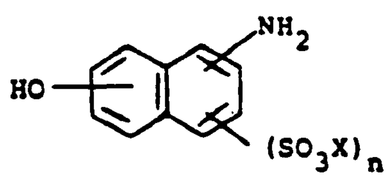

en un composé N-acylaminé de formule

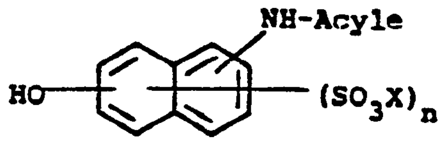

on fait ensuite réagir ce composé avec des halogénures d'acide sulfonique de formule

$$R\text{-}SO_2Y$$

dans laquelle

Y est un halogène, de préférence le chlore pour obtenir les composés

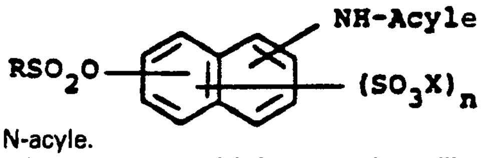

et on élimine ensuite le reste N-acyle.

2. Procédé suivant la revendication 1, caractérisé en ce qu'on utilise pour N-acylation des anhydrides de diacides carboxyliques aliphatiques ou aromatiques.

3. Procédé suivant les revendications 1 et 2, caractérisé en ce qu'on opère sans isolement intermédiaire.

**Claims**

1. Process for the preparation of esters of the formula

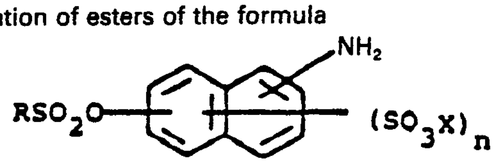

wherein

X = H or a monovalent cation,
R = alkyl, aralkyl or aryl and
n = 1 or 2,

characterised in that first of all aminonaphthols of the formula

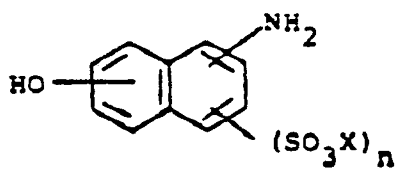

are converted into an N-acylamino compound of the formula

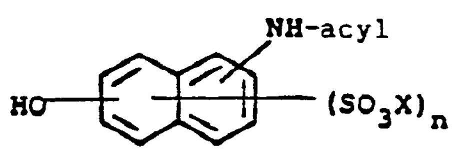

which is then reacted with sulphonic acid halides of the formula

$$R\text{-}SO_2Y$$

wherein

Y = halogen, preferably Cl,

to give the compounds

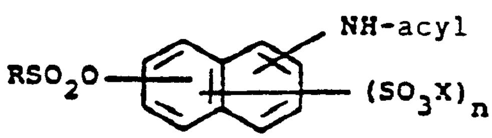

and then the N-acyl radical is split off.

2. Process according to Claim 1 characterised in that anhydrides of dibasic aliphatic or aromatic carboxylic acids are used for the N-acylation.

3. Process according to Claims 1 and 2, characterised that it is carried out without intermediate isolation.